**Europäisches Patentamt**

(19) **European Patent Office**

**Office européen des brevets**

(11) Veröffentlichungsnummer: **0 008 105**
**B1**

## EUROPÄISCHE PATENTSCHRIFT

(45) Veröffentlichungstag der Patentschrift:
03.03.82

(51) Int. Cl.³: **A 61 K 7/00**

(21) Anmeldenummer: **79102826.9**

(22) Anmeldetag: **06.08.79**

(54) **Verwendung von Citronensäureestern in kosmetischen Zubereitungen.**

(30) Priorität: **08.08.78 DE 2834645**

(43) Veröffentlichungstag der Anmeldung:
**20.02.80 Patentblatt 80/4**

(45) Bekanntmachung des Hinweises auf die Patenterteilung:
**03.03.82 Patentblatt 82/9**

(84) Benannte Vertragsstaaten:
**AT BE CH DE FR GB IT NL SE**

(56) Entgegenhaltungen:
**DE-A-1 952 057**
**DE-A-2 452 119**
**DE-A-2 418 338**
**DE-B-1 286 692**

**H. JANISTYN: «Handbuch der Kosmetika und Riechstoffe», Vol. 1, «Die Kosmetischen Grundstoffe»
1969, Hüthig Verlag Heidelberg, Seite 228, Deutsch-
land**

**CHEMICAL ABSTRACTS, Vol. 83, Nr. 22, 1. Dezember
1975, Seite 265, Spalte 1, Nr. 183 303g, Columbus,
Ohio, U.S.A.**

**Die Akte enthält technische Angaben, die nach dem
Eingang der Anmeldung eingereicht wurden und die nicht
in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Stork, Karl, Dr. Dipl.-Chem., in der Gewann 5,
D-6840 Lampertheim (DE)**
Erfinder: **Oppenlaender, Knut, Dr. Dipl.-Chem.,
Otto-Dill-Strasse 23, D-6700 Ludwigshafen (DE)**
Erfinder: **Selb, Karl, Dr. Dipl.-Chem., Kriemhildstrasse 36,
D-6940 Weinheim (DE)**
Erfinder: **Naegele, Paul, Wiesenstrasse 9,
D-6701 Neuhofen (DE)**

Verwendung von Citronensäureestern in kosmetischen Zubereitungen

Die Erfindung betrifft die Verwendung von Citronensäureisoalkylestern mittlerer Kettenlänge als flüssige Ölkomponente in kosmetischen Zubereitungen, die sich durch besonders günstiges Löslichkeitsverhalten und günstiges Viskositätsverhalten auszeichnen. Die damit hergestellten Emulsionen bleiben über einen längeren Zeitraum stabiler als mit Vergleichsprodukten.

Aus der Literatur ist die Verwendung von Citronensäureestern auf diesem Gebiet schon bekannt. Die veröffentlichten japanischen Patentanmeldungen JP-A-75/63145 und 75/82104 lehren Citronensäurealkylester mit mehr als 16 C-Atomen in der Alkoholkomponente als Basis für kosmetische Öle.

Diese Verbindungen sind z.B. in Alkohol/Wasser-Gemischen unlöslich und weisen zu hohe Stockpunkte und ein schlechtes Viskositätsverhalten auf.

Aus DE-A 2 418 338 sind kosmetische Mittel bekannt, die Citronensäure und/oder Acetylcitronensäureester mit 1 bis 6 C-Atomen in der Alkoholkomponente enthalten.

Diese Produkte weisen wegen ihres höheren Dampfdruckes einen relativ starken Eigengeruch auf, der die Duftnote beispielsweise von parfümierten Cremes stört, und sind zudem schlecht öllöslich.

Aus K. Rothmann, 4. Auflage, 1969, Dr. Alfred Hüthig Verlag, Heidelberg, «Das grosse Rezeptbuch der Haut- und Körperpflegemittel», Seite 146, ist bekannt, z.B. Citronensäureoctylester in Körperpflegemitteln einzusetzen. Dieser einen geradkettigen Alkoholrest enthaltende Ester weist aber eine zu hohe Viskosität auf, so dass er für viele Zwecke (Körpermilch, Rasierwasser usw.) ungeeignet ist.

Aus dem «Handbuch der Kosmetika und Riechstoffe» von Hugo Janistyn, 2. Aufl., Seite 228, ist Acetyltri-2-ethyl-hexylcitrat («Citroflex A 8») bekannt. Es wird an dieser Stelle angegeben, dass die bewusste Verbindung z.B. als Weichmacher in Nagellacken zur Anwendung gelangt, d.h. nicht als Ölkomponente in Cremes oder sonstigen kosmetischen Zubereitungen. Das Acetylderivat ist ausserdem in Wasser/Alkohol-Gemischen schlecht löslich und bewirkt weniger stabile Emulsionen. Aus DE-B 1 286 692 sind kosmetische Präparationen bekannt, die – beispielhaft ist das nicht belegt – u.a. Alkylester der Citronensäure enthalten können. Diese Mittel sollen speziell die Wasserdampfdurchlässigkeit der Cremes auf der Haut erhöhen; als reine Ölkomponente sind sie nicht geeignet, da sie wegen der noch freien Carboxylgruppen in der Ölphase nicht mehr genügend löslich sind.

Ziel der Erfindung ist es, eine Ölkomponente in kosmetischen Zubereitungen zu finden, die ungiftig ist, günstige Viskositäten aufweist, öllöslich und löslich in Alkohol/Wasser-Mischungen und emulgierbar ist; die damit hergestellten Wasser-in-Öl- und Öl-in-Wasser-Emulsionen sollen gleichermassen stabil sein. Ferner soll die Ölkomponente auch bei höherer Sommertemperatur – also bei 40 °C oder mehr – und zudem in heissem Badewasser geruchlos sein.

Dieses Ziel wird mit Citronensäureestern erreicht, die als Alkoholreste verzweigtkettige 8 bis 13 C-Atome aufweisende Alkoholreste tragen.

Als Alkohole für die Veresterung mit der Citronensäure kommen alle verzweigtkettigen Vertreter obiger Definition in Betracht, beispielsweise 2-Äthylhexanol (Isooctanol), Isononanol, Isodecanol und Isotridecanol sowie deren Mischungen.

Die letzteren Bezeichnungen für die Alkohole stellen Trivialbezeichnungen für nach der Oxosynthese erhaltene Alkohole dar – siehe Ullmann «Encyklopädie der technischen Chemie», 3. Auflage, Ergänzungsband, Seite 99 unten bis Seite 100 oben.

Die erfindungsgemäss zu verwendenden Ester können in vielfältigen kosmetischen Zubereitungen als flüssige Ölkomponente dienen, was seine Ursache in ihren ausgezeichneten Eigenschaften bezüglich Löslichkeit in Öl, Wasser-Alkohol-Gemischen, bezüglich ihrer Emulgierbarkeit und Emulsionsstabilität in Emulsionen des Öl-Wasser- und Wasser-Öl-Typs, bezüglich ihrer günstigen Viskositäten und ihrer Geruchsfreiheit bei den in der Kosmetik üblichen Anwendungstemperaturen hat.

Solche Präparationen sind z.B. Öl-in-Wasser-Cremes, die im allgemeinen aus einer Ölphase (Vaseline, Paraffinöl, native und synthetische Triglyceride, Esteröle, Lanolin, Silikonöle, Glycerinmono- und -distearat, Emulgatoren) und einer Wasserphase bestehen, oder Wasser-in-Öl-Cremes, die dieselben Bestandteile enthalten, jedoch umgekehrt emulgiert sind. Einen ähnlichen Aufbau weist auch eine Reinigungsmilch auf; es sind flüssige Cremes.

Weitere Präparationen sind Rasierwässer, die Alkohol-Wasser-Gemische darstellen, und die Bactericide, organische Säuren, Adstringentien und Überfettungsmittel enthalten. Schliesslich gehören auch Badezusätze zu solchen Präparationen, die Tenside und Überfettungsmittel enthalten.

Alle diese Präparationen enthalten erfindungsgemäss die Citronensäureester in Mengen von 4 bis 10 Gewichtsprozent, bezogen auf die gesamte Präparation. Die Mengen richten sich nach der Natur der einzelnen Präparationen. Produkte mit überwiegenden Mengen an Ölen enthalten ca. 20 bis 40 Gewichtsprozent an Citronensäureestern (Badeöle, Körperöle); Produkte mit höherem Wasser- oder Alkohol/Wasser-Gehalt enthalten 4 bis ca. 20 Gewichtsprozent (Cremes, Rasierwässer, Körpermilch).

Die nun folgenden Beispiele geben Rezepte wieder, welche die erfindungsgemäss zu verwendenden Ester empfehlen.

Beispiel 1
Creme Öl/Wasser (Gew.-%)
Glycerinmonostearat 8,0
Cetylalkohol 2,0
$C_{16}/C_{18}$ Alkohol, 25fach äthoxyliert 2,0
$C_{16}/C_{18}$ Alkohol, 6fach äthoxyliert 2,0
Citronensäure-tris-isonomylester 15,0
1,2-Propylenglykol 3,0
Konservierungsmittel 0,5
Parfümöl 0,2
Wasser 67,3

Beispiel 2
Creme Wasser/Öl (Gew.-%)
Vaseline 20,0
Citronensäure-tris-isodecylester 5,0
Oleyldiglycerinäther 2,0
Cholesterin 0,5
Mikrokristallines Wachs 2,0
1,2-Propylenglykol 3,0
Magnesiumsulfat-hepta-hydrat 0,6
Konservierungsmittel 0,5
Parfümöl 0,3
Wasser 66,1

Beispiel 3
Reinigungsmilch Öl/Wasser (Gew.-%)
Glycerinmonostearat 3,0
$C_{16}/C_{18}$ Alkohol, 11fach äthoxyliert 2,0
$C_{16}/C_{18}$ Alkohol, 6fach äthoxyliert 2,0
Paraffinöl 10,0
Citronensäure-tris-2-äthylhexylester 10,0
Erdnussöl 5,0
1,2-Propylenglykol 3,0
Konservierungsmittel 0,5
Parfümöl 0,2
Wasser 64,3

Beispiel 4
Rasierwasser (Gew.-%)
Äthanol 80 Gew.-% 93,4
Citronensäure-tris-isononylester 5,0
Parfümöl 0,5
1,2-Propylenglykol 1,0
Menthol krist. 0,1

Beispiel 5
Glycerincreme Öl/Wasser (Gew.-%)
Glycerinmonostearat 8,0
Cetylalkohol 2,0

(Gew.-%)
$C_{16}/C_{18}$ Alkohol, 25fach äthoxyliert 2,0
Glycerin 20,0
Citronensäure-tris-isotridecylester 8,0
Silikonöl 350 1,0
Konservierungsmittel 0,5
Parfümöl 0,2
Wasser 5,3

Beispiel 6
Körperöl (Gew.-%)
Paraffinöl 60,0
Capryl/Caprinsäuretriglycerid 20,0
Citronensäure-tris-isodecylester 20,0

Beispiel 7
Badeöl für Cremebad (Gew.-%)
Rezept 1
Paraffinöl 60,0
Citronensäure-tris-isotridecylester 35,0
Oleyldiglycerinäther + 2 Mol Äthylenoxid 5,0

Rezept 2
Paraffinöl 50,0
Olivenöl 10,0
Citronensäure-tris-isononylester 35,0
Oleyldiglycerinäther + 2 Mol Äthylenoxid 5,0

**Patentanspruch**

Verwendung von Estern der Citronensäure mit verzweigtkettigen aliphatischen Alkoholen mit 8 bis 13 Kohlenstoffatomen pro Alkoholrest als flüssige Ölkomponente in kosmetischen Zubereitungen.

**Claim**

Use of esters of citric acid with branched-chain aliphatic alcohols of 8 to 13 carbon atoms as liquid oil component in cosmetic preparations.

**Revendication**

Utilisation d'esters de l'acide citrique et d'alcools aliphatiques à chaîne ramifiée avec 8 à 13 atomes de carbone par reste alcool comme composante huileuse liquide dans des compositions cosmétiques.